# EUROPEAN PATENT SPECIFICATION

(11) **EP 1 137 450 B1**
(45) Date of publication and mention of the grant of the patent: **23.08.2006**
(21) Application number: 00968400.2
(22) Date of filing: 06.10.2000
(51) Int. Cl.: A61M 5/00, A61M 5/19

(54) **APPARATUS FOR HOLDING AND OPERATING ONE OR MORE SYRINGES**
GERÄT ZUM HALTEN UND BETÄTIGEN EINER ODER MEHRERER SPRITZEN
APPAREIL PERMETTANT DE MAINTENIR ET DE FAIRE FONCTIONNER UNE OU PLUSIEURS SERINGUES

(30) Priority: 08.10.1999 US 158302 P
(43) Date of publication of application: 04.10.2001
(73) Proprietor: Harvest Technologies Corporation, Plymouth, Massachusetts 02360 (US)
(72) Inventor: VERKAART, Wesley, H., Duxbury, MA 02332 (US)
(74) Representative: Crouch, David John
(86) International application number: PCT/US2000/026243
(87) International publication number: WO 2001/026712

(56) References cited:
- EP-A- 0 538 174
- WO-A-98/40115
- US-A- 3 016 897
- US-A- 4 979 942
- US-A- 5 179 983
- US-A- 5 290 259
- US-A- 5 395 326
- US-A- 5 582 596

## Description

### TECHNICAL FIELD

This invention relates to syringes. In particular, the invention relates to a device for holding one or more syringes to facilitate operation of a single syringe or to operate and combine the fluids from a plurality of syringes.

### BACKGROUND ART

The operation of a syringe is well known. In a common use, a hypodermic needle is attached to the barrel of a syringe for injecting a fluid into a patient. Syringes also have other uses, such as the application of a fluid to an exterior surface or the injection of a fluid into a conduit.

It is also known to combine the fluids from two separate syringes for application of the mixture to an object. An example of this is the combination of a first fluid containing fibrinogen in a first syringe and a second fluid containing thrombin in a second syringe to provide a fibrin sealant. This type of syringe typically provides a combining tip having two inlets, each of which receives the outlet of a respective one of two syringes. The combining tip provides a Y-type channel for combining the two fluids.

A problem in this art is how to facilitate handling the syringes and, in particular, how to handle two or more syringes such that they can be operated simultaneously with ease. In this regard, it is often desirable to link the syringe barrels and the syringe plungers to allow the operator to apply the contents in a controlled manner with one hand.

It may also be desirable to provide for operation of a single syringe by using the thumb to obtain more leverage on the end of the plunger.

A further requirement of devices in this art is that the components be inexpensive, easily sterilized, and disposable.

### SUMMARY OF THE INVENTION

The present invention is directed to apparatus for holding and operating two or more syringes as set out in the accompanying claim 1. Preferred features are set out in sub-ordinate claims 2 to 9. A document disclosing the preamble of claim 1 is EP 0 538 174.

In accordance with the invention, a one-piece molded plastic handle receives the barrel of a first syringe for securely holding it in a position whereby the user may grip the handle and operate the plunger with the thumb. A clip is also provided for connecting the plunger of a syringe placed in the handle to the plunger of one or more additional syringes.

In use, the outlet ends of the syringes are attached to any of several applicator tips that are commercially available for, combining fluids from two or more syringes. Thus, luer lock tips at the outlet ends of the syringes can be are secured to the applicator tip while the plungers at the opposite ends are secured to the clip provided by the invention to result in a rigid assembly, with the syringe barrels essentially parallel.

One of the syringes is held in the handle so that the operator can operate the syringes simultaneously with the thumb by grasping the handle and pushing on the clip with the thumb.

The syringes may be of the same size, which would provide a 1: 1 mixture ratio of the fluids. Or, the syringes may be of different sizes to provide another desired mixing ratio. For example, if the first syringe has a 10ml capacity, and a second syringe has a 1ml capacity, the mixing ratio will be 10:1. Syringes of different sizes are easily accommodated because the handle holds only a single syringe, and the remaining syringes are held to the first by the applicator and the clip. By this construction, different sizes of syringes can accommodated with a single handle by using a clip specifically designed to engage the plungers of the syringes. The preferred handle includes a cradle sized to receive the barrel of a 10ml syringe. The cradle is open at the top so the barrel can be snapped into the handle from the top or slid in from either end. The cradle can be a cylinder, which may require an adaptor for receiving barrels of different sizes.

The handle also preferably provides a storage bay for one or more clips, which may be of different sizes or may be redundant in case one is dropped during assembly.

In the preferred embodiment, the handle and the slips are injection-molded polypropylene of a grade that withstands sterilization by irradiation or ethylene oxide gas

### BRIEF DESCRIPTION OF THE DRAWINGS

Figure 1 is a side view of an apparatus for holding one or more syringes in accordance with the invention.
Figure 2 is a side view of the apparatus of figure 1 with an applicator tip attached to the syringes.
Figure 3 is a side view of an apparatus in accordance with the invention with an alternate clip.
Figure 4a is an end view of a preferred embodiment of a handle in accordance with the invention.
Figure 4b is an end view of an alternate embodiment of a handle in accordance with the invention.
Figure 5a is a side view of a handle showing storage of the clips.
Figure 5b is a perspective of the embodiment of figure 5a.
Figure 6 is a side view of the embodiment shown in figure 4b showing an adaptor for accommodating syringes of different sizes.

### DETAILED DESCRIPTION OF THE PREFERRED EMBODIMENT

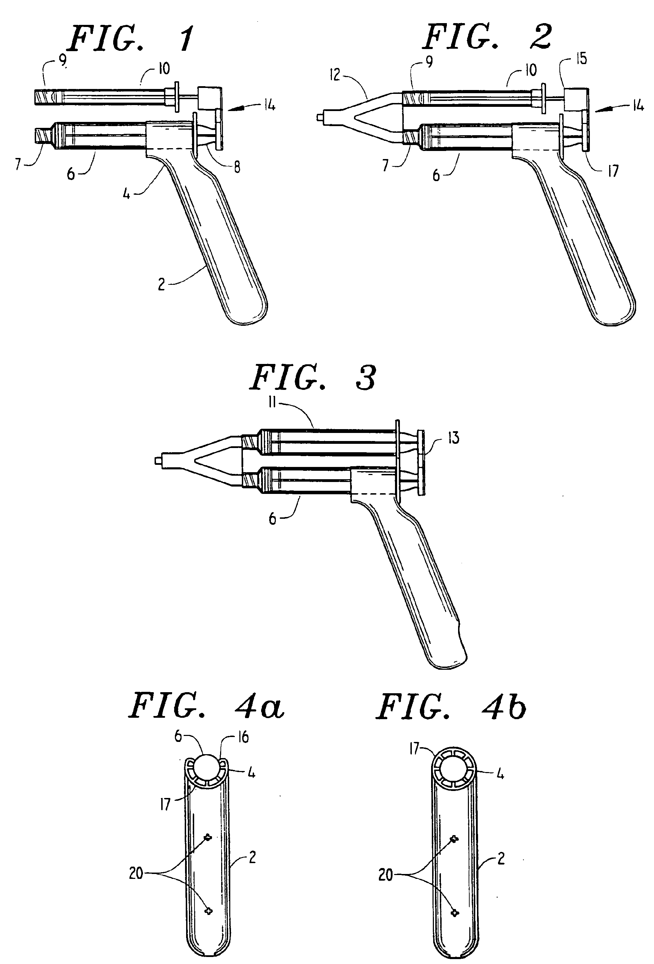

With reference to figure 1, a preferred embodiment of the invention includes a handle 2, which is designed with a cradle portion 4 for receiving a first syringe 6. The handle provides a grip for engaging the palm of a user in such a configuration that the thumb of the user is positioned to engage easily the plunger 8 of the syringe 6. A second syringe 10 is held parallel to the first syringe by structure to be described. Each synnge has a luer lock 7 and 9, respectively, for connecting the syringes to conduits, or the like.

With reference to figure 2, an applicator tip 12 is shown connected to the luer lock ends of the first and second syringes 6 and 10. This applicator is one of several known in the art for combining the fluids from the first and second syringes to provide a mixture of the fluids from the two syringes. More than two syringes may be employed with an appropriate applicator tip.

Figure 2 also shows use of a clip 14, which is secured to the ends of the syringe plungers such that a user can operate the syringes simultaneously by pressing on the clip 14. The clip 14 can be stepped to provide attachment pans of different thickness to accommodate syringes of different lengths. Thus, the clip shown in figure 2 provides a first portion 15 that is thicker than a second portion 17 by the difference between the lengths of the two syringes 6 and 10. Figure 3 shows an alternate clip 13 of uniform thickness, to be used when the syringes are of the same length.

Figure 4a is an end view of a handle 2 having a cradle 4 designed to receive a syringe barrel either by insertion from above the cradle in a downward direction or by longitudinally sliding the syringe barrel into the cradle. In this embodiment, the sides 16 of the cradle project inward and downward slightly and are resilient to receive the barrel of a syringe 6 and hold it securely to the handle. Radial ribs 17 are spaced about the cradle 4 for engaging the syringe and providing secure support by the cradle. In this embodiment, the barrel can be inserted in a direction transverse to the longitudinal axis of the barrel or slid longitudinally into the barrel.

Figure 4b shows an alternate embodiment wherein the cradle is designed to receive the barrel only by sliding it longitudinally into the cradle.

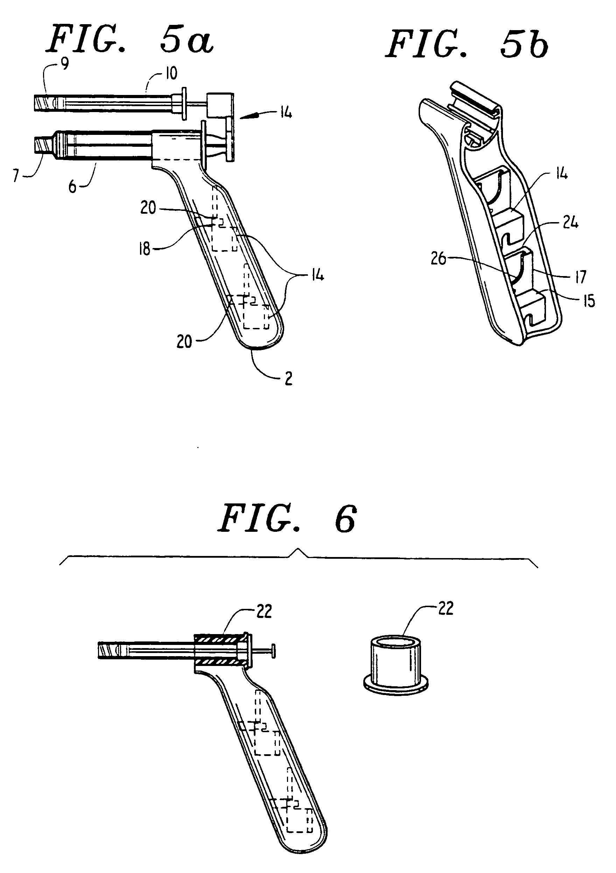

Figure 5a is a side view of the invention showing how spare clips 14 are stored in an open cavity formed by the sides of the handle. In the preferred embodiment, each of the clips has an opening 18 for receiving a post 20 on the handle to hold the clip to the handle such that a user can readily remove the clip from its storage position. The post is preferably cruciform in cross section for resiliently holding the clip.

Figure 5b is a perspective showing two clips stored in the handle. It will be appreciated that each clip includes front and back walls forming two adjacent slots 24, each of which receives a flange on the end of a respective syringe plunger. The front wall is cut out at 26 to accept the plunger's shaft. The slots are preferably directed oppositely, whereby the plungers are inserted from opposite ends of the clip to accommodate the natural tendency of the syringes to come toward each other.

Figure 6 shows how a handle, such as that shown in figure 4b, can be adapted to receive syringes of different sizes. In this embodiment, an adaptor 22 is provided for altering the size of the cradle to receive a syringe having a diameter smaller than the largest nominal size to be retained by the cradle. A plurality of these adaptors may be provided to accommodate syringes of various sizes.

In use, the syringes may be assembled in almost any order. In one procedure, the applicator tip is secured to the syringes, the syringe 6 is inserted into the cradle 4, and the clip attached. Alternatively, the syringe 6 is inserted into the handle, and the applicator tip, second syringe and clip are then attached. The parts may be assembled in other orders also.

It will be appreciated that a novel apparatus for holding and operating one or more syringes has been described. Modifications within the scope of the appended claims will be apparent to those of skill in the art.

## Claims

1. Apparatus for holding and operating two or more syringes (6, 10) each of which has a barrel portion and a plunger (8), comprising a handle (2) extending generally along a handle longitudinal axis and having a portion configured to be gripped in a hand of a user and a cradle at one end of the handle extending generally along a cradle longitudinal axis, **characterised in that** the cradle, in use removably engages and holds in position a barrel portion of a single syringe of the two or more syringes (6, 10) such that the cradle longitudinal axis intersects and extends transversely to the handle (2) longitudinal axis and is located such that a plunger of the single syringe engaged in the cradle is easily engaged by the thumb of the hand of a user, and a clip (14) adapted in use to engage only the ends of the plungers (8) of at least two of said syringes (6,10) and to connect the plungers (8) for simultaneous movement.

2. Apparatus according to claim 1 **characterised in that** the clip accommodates syringes of different lengths.

3. Apparatus according to claim 1 or claim 2 **characterised in that** the apparatus further comprises an applicator tip for connecting to the output end of each syringe and receiving the outputs from the syringes.

4. Apparatus according to any one of claims 1 to 3 **characterised in that** the handle provides a cavity for storing and readily removing one or more of the clips.

5. Apparatus according to claim 4 **characterised in that** the handle includes at least one post for engaging an opening in the clip.

6. Apparatus according to any preceding claim **characterised in that** the clip comprises spaced front and rear walls forming grooves to receive the ends of the plungers.

7. Apparatus according to claim 6 **characterised in that** one of the walls is cut out to receive the shaft of a syringe plunger.

8. Apparatus according to any preceding claim **characterised in that** the cradle is semi-cylindrical and is made of resilient material for receiving the said barrel portion of the said syringe when inserted in a direction toward said handle portion.

9. Apparatus according to any preceding claim **characterised in that** the apparatus further comprises an adaptor configured to adapt the cradle to engage a syringe barrel having a transverse dimension smaller than that of the cradle.

## Patentansprüche

1. Vorrichtung zum Halten und Bedienen zweier oder mehrerer Spritzen (6, 10), wovon jede einen Zylinderabschnitt und einen Kolben (8) umfasst, mit einem Handgriff (2), der sich allgemein entlang einer Handgrifflängsachse erstreckt und einen Abschnitt, der dazu ausgelegt ist, in der Hand eines Benutzers festgehalten zu werden, und eine Halterung an einem Ende des Handgriffs aufweist, die sich allgemein entlang einer Halterungslängsachse erstreckt, **dadurch gekennzeichnet, dass** die Halterung im Gebrauch einen Zylinderabschnitt einer einzelnen der beiden oder mehreren Spritzen (6, 10) derart abnehmbar in Eingriff nimmt und an Ort und Stelle hält, dass die Halterungslängsachse die Längsachse des Handgriffs (2) kreuzt und sich quer dazu erstreckt und so angeordnet ist, dass ein Kolben der einzelnen Spritze, die in der Halterung in Eingriff ist, mühelos mit dem Daumen der Hand eines Benutzers in Eingriff genommen werden kann, und eine Klammer (14) dazu ausgelegt ist, im Gebrauch nur die Enden der Kolben (8) von mindestens zweien der Spritzen (6, 10) in Eingriff zu nehmen und die Kolben (8) für eine gleichzeitige Bewegung zu verbinden.

2. Vorrichtung nach Anspruch 1, **dadurch gekennzeichnet, dass** die Klammer verschieden lange Spritzen aufnimmt.

3. Vorrichtung nach Anspruch 1 oder Anspruch 2, **dadurch gekennzeichnet, dass** die Vorrichtung darüber hinaus eine Applikatorspitze umfasst, um an das Abgabeende jeder Spritze angeschlossen zu werden und die Abgaben aus den Spritzen aufzunehmen.

4. Vorrichtung nach einem der Ansprüche 1 bis 3, **dadurch gekennzeichnet, dass** der Handgriff einen Hohlraum zur Aufbewahrung und mühelosen Entnahme einer Klammer oder mehrerer Klammern bereitstellt.

5. Vorrichtung nach Anspruch 4, **dadurch gekennzeichnet, dass** der Handgriff mindestens einen Stiel umfasst, um eine Öffnung in der Klammer in Eingriff zu nehmen.

6. Vorrichtung nach jedem vorhergehenden Anspruch, **dadurch gekennzeichnet, dass** die Klammer beabstandete Vorder- und Rückwände umfasst, die Nuten bilden, um die Enden der Kolben aufzunehmen.

7. Vorrichtung nach Anspruch 6, **dadurch gekennzeichnet, dass** eine der Wände ausgeschnitten ist, um den Schaft eines Spritzenkolbens aufzunehmen.

8. Vorrichtung nach jedem vorhergehenden Anspruch, **dadurch gekennzeichnet, dass** die Halterung halbzylindrisch ist und aus einem nachgiebigen Material besteht, um den Zylinderabschnitt der Spritze beim Einführen in einer Richtung zum Handgriff hin aufzunehmen.

9. Vorrichtung nach jedem vorhergehenden Anspruch, **dadurch gekennzeichnet, dass** die Vorrichtung darüber hinaus ein Anpassstück umfasst, das dazu ausgelegt ist, die Halterung dazu anzupassen, einen Spritzenzylinder mit einer Querabmessung in Eingriff zu nehmen, die kleiner ist als diejenige der Halterung.

## Revendications

1. Appareil pour maintenir et actionner deux ou plusieurs seringues (6, 10) dont chacune a une partie de corps cylindrique et un piston plongeur (8), comprenant une poignée (2) s'étendant généralement le long d'un axe longitudinal de poignée et ayant une partie configurée pour être saisie dans une main d'un utilisateur et un berceau au niveau d'une extrémité de la poignée s'étendant généralement le long d'un axe longitudinal de berceau, **caractérisé en ce que** le berceau, à l'usage, met en prise de manière amovible et maintient en position une partie de corps cylindrique d'une seule seringue des deux ou plusieurs seringues (6, 10) de sorte que l'axe longitudinal de berceau coupe et s'étend transversalement par rapport à l'axe longitudinal de la poignée (2) et est positionné de sorte qu'un piston plongeur de la seringue unique mise en prise dans le berceau est mis en prise facilement par le pouce de la main d'un utilisateur, et une attache (14) adaptée à l'usage pour ne mettre en prise que les extrémités des pistons plongeurs (8) d'au moins deux desdites seringues (6, 10) et pour raccorder les pistons plongeurs (8) pour le mouvement simultané.

2. Appareil selon la revendication 1, **caractérisé en ce que** l'attache accepte des seringues de différentes longueurs.

3. Appareil selon la revendication 1 ou la revendication 2, **caractérisé en ce que** l'appareil comprend en outre une pointe d'applicateur pour raccorder l'extrémité de sortie de chaque seringue et recevoir les sorties provenant des seringues.

4. Appareil selon l'une quelconque des revendications 1 à 3, **caractérisé en ce que** la poignée propose une cavité pour stocker et retirer facilement une ou plusieurs des attaches.

5. Appareil selon la revendication 4, **caractérisé en ce que** la poignée comprend au moins un montant pour mettre en prise une ouverture dans l'attache.

6. Appareil selon l'une quelconque des revendications précédentes, **caractérisé en ce que** l'attache comprend des parois avant arrière espacées formant des rainures pour recevoir les extrémités des pistons plongeurs.

7. Appareil selon la revendication 6, **caractérisé en ce que** l'une des parois est coupée pour recevoir la tige d'un piston plongeur de seringue.

8. Appareil selon l'une quelconque des revendications précédentes, **caractérisé en ce que** le berceau est semi-cylindrique et est réalisé avec un matériau élastique pour recevoir ladite partie de corps cylindrique de ladite seringue lorsqu'elle est insérée dans une direction allant vers ladite partie de poignée.

9. Appareil selon l'une quelconque des revendications précédentes, **caractérisé en ce que** l'appareil comprend en outre un adaptateur configuré pour adapter le berceau afin de mettre en prise un corps cylindrique de seringue ayant une dimension transversale inférieure à celle du berceau.
